# EUROPEAN PATENT APPLICATION

(11) **EP 3 473 277 A1**
(43) Date of publication of application: **24.04.2019**
(21) Application number: 18159860.8
(22) Date of filing: 05.03.2018
(51) Int. Cl.: A61L 27/16, A61L 27/32

(54) **METHOD FOR BIOMIMETIC GROWTH OF CALCIUM PHOSPHATES CERAMICS ON METAL IMPLANTS**

(30) Priority: 20.10.2017 EP 17197488
(71) Applicant: PAUL SCHERRER INSTITUT, 5232 Villigen PSI (CH)
(72) Inventor: CARINO, Agnese, 5417 Untersiggenthal (CH); TESTINO, Andrea, 5417 Untersiggenthal (CH)
(74) Representative: Fischer, Michael

(57) **Abstract**

The objective of the present invention to provide a method for growth of calcium phosphates ceramics (CPs) on metal implants, particularly on Ti and Ti-alloy implants, that generates thin and durable coatings of metal implants without the limitation of other techniques known in the art. This objective is achieved according to the present invention by a biomimetic method for which comprises the following steps:
a) a combination of mechanical, chemical, and thermal treatments in order to generate an appropriate grafting layer on the metal implant; and
b) a coating procedure of the treated metal implant by controlled CaPc precipitation.

The results demonstrated that metal implants can be efficiently coated with CaPc in a short time using a wet method, offering unprecedented opportunities. This wet route is carried out at almost room temperature, at low cost, with an environmental-friendly method with low energy consumption, and in a short time. According to the invention, an innovative and extremely competitive method for coating of metal implants with CaPc is claimed.

## Description

The present invention relates to a method for biomimetic growth of calcium phosphates ceramics on metal implants, particularly on Ti and Ti-alloy implants.

Parallel to the development of economy and technology, the number of aged people demanding compromised tissue replacement is rapidly increasing. Moreover, there is an expanding requirement for hard tissue replacement as a consequence of sports related and degenerative injuries. It is estimated that >70% of biomedical implants are made of metallic materials. Among them, Ti and its alloys are widely used in dental, craniomaxillofacial, spinal and orthopaedic surgery because of their excellent mechanical properties that are required for load bearing applications.

Titanium implants can be osseointegrated; that is the structural and functional connection between bone tissue and the surface of an artificial implant. This phenomenon was discovered in 1952 by the Swedish professor Per-Ingvar Branemark - considered the father of the modern dental implantology - who found that titanium attaches itself to the bone when it is implanted in it. The osseointegration is possible thanks to the native thin layer of TiO₂ on the Ti surface. The topography and chemistry of the metal surface is usually modified to improve its biocompatibility and bioactivity: as the interaction between the cells and tissues with biomaterials is a surface phenomenon occurring at the tissue-implant interface, implant surface properties play a major role in determining both the biological response to implants and the material response to the physiological conditions.

Several studies were carried out in order to improve osseointegration and it was concluded than an optimal surface roughness of the metal implants, which is in the range of few micrometers, offer clinical advantages. This roughness is generally obtained by blasting or electrochemical methods. Additional surface treatments may be applied, such as acidic etching, in order to further promote the natural bone growth *in vivo.*

A further improvement is achievable if the implant is coated with bone-like ceramic materials, such as calcium phosphate ceramics (CaPc), which are promoting a much faster osseointegration.

Several CaPc exist. The most commonly used is hydroxyapatite (HA), but alternative materials, such as octacalcium phosphate (OCP), dicalcium phosphate dihydrate (DCPD), tricalcium phosphate (TCP), and dicalcium phosphate anhydrous (DCPA) might be applied.

HA is mainly deposited by plasma-spraying technique directly onto Ti substrate. Plasma-spray, although clinically established, has specific drawbacks such as the extremely high processing temperatures and, as for other spray techniques, difficulties to coat complex-shaped parts due to the line of sight issues. Alternatively, CaPc can be deposited by electrochemical deposition (ECD) methods: typically DCPD or HA is deposited in solution with this technique. ECD offers advantages because no high temperature is applied and some line of sight difficulties are solved. Nevertheless, the non-homogeneous electric field in solution produces areas where the coating is rather thick and other zones where no deposition occurs due to shielding effects. Moreover, thin coating capability remains a major limitation.

Recently the discrete particle deposition (DPD) method has been reported. Here, the implant is immersed in a suspension of CaPc particles, typically HA nanoparticles, and some of those particles remain on the implant surface. This method, known in several others fields as "dip coating", allows the fast and easy materials deposition on the substrate but the interaction between the particles and the substrate is rather labile. Moreover, the surface can be only partially coated. Nevertheless, even this simple surface deposition was reported to be beneficial for faster osseointegration.

To overcome some limitations of the aforementioned deposition techniques, wet or biomimetic methods were studied. Most of the reported wet method approaches consist of soaking the implant in a simulated body fluid (SBF) solution for several days and let the CaPc grows on the implant surface. The main disadvantage of this method is the long time required for coating which makes the process not commercially valuable.

One of the main concerns about ceramic coatings, in particular for dental implants, is related to delamination, that is the disconnection between the metal implants and the ceramic coating. In case of delamination, inflammation occurs, and the implant fails. Delamination can be avoided by an appropriate metal-ceramic joining and by controlling the CaPc thickness. Furthermore, the disadvantage of a thick coating is that the micro-topography of the implant surface and the consequent beneficial topographical effect of the surface morphology might be lost.

In particular, in the field of dental implants, the stability of the implants is a key feature. Just after the implantation, a primary stability (or mechanical stability) is attained; this mainly depends on the design of the screw, the surgical technique, and the bone conditions. After a longer time, e.g., three-six months, osseointegration occurs and the so-called secondary stability (or biological stability) is attained. The overall implant stability is the sum of primary and secondary stabilities, which shows a minimum 2-4 weeks after the implantation. During this timeframe, the probability of implant failure is maximized. Rapid osseointegration (e.g. a short healing time) shifts the overall implant stability towards a shallower minimum, thereby decreasing the implant failure probability and reducing the unpleasant patient period between surgery and a normal chewing performance. CaPc coating on dental implants can play a relevant beneficial role by shortening the healing time provided that the aforementioned issues associated to the deposition method are solved.

It is therefore objective of the present invention a method for CaPc coating of metal implants, particularly of Ti and Ti alloy implants, that provide a thin CaPc coating which is strongly bonded to the metal implants and deposited in a short time, thus without the limitations and drawbacks of the techniques currently known in the art.

This objective is achieved according to the present invention by a biomimetic method for CaPc coating of metal implants, particularly on Ti and Ti alloy implants, comprising the following steps:
Step-1: a combination of treatments in order to generate an appropriate grafting layer on the metal implant; such Step-1 is preferably composed of one or more of the following Sub-process:
   i) Sub-process-1: a mechanical or electrochemical treatment on the machined surface in order to generate a primary roughness in the range of few micrometers; and/or
   ii) Sub-process-2: a chemical treatment with an alkaline solution, in order to generate an appropriate chemical phase on the surface of the metal implant; and/or
   iii) Sub-process-3: a thermal treatment in order to promote an appropriate metal-ceramic joining of the grafting layer; and/or
   iv) Sub-process-4: a final chemical treatment with an alkaline solution in order to improve the reactivity grafting layer; and
Step-2: the implant treated according to Step-1 is coated with CaPc by controlled precipitation.

The results demonstrated that metals, in particular, Ti and Ti alloy, can be efficiently coated with CaPc in a short time using a wet method, offering unprecedented opportunities. This wet route is carried out at almost room temperature, at low cost, with an environmental-friendly method with low energy consumption, and in a short time. According to the invention, an innovative and extremely accurate method for metal implant coating with CaPc has been developed. Moreover, the present results carried out in controlled precipitation conditions are showing efficient control over the CaPc phase deposited. In particular, OCP can be deposited. *In vivo* experiments have shown that OCP is rapidly converted to HA within 7-10 days after implantation and the rate of new bone formation on HA converted from OCP was much greater than that of both calcium-deficient HA and stoichiometric HA implanted directly. The effect of calcium phosphates on the osteoblastic activity and bone regeneration, with particular emphasis on OCP activity, has been recently reviewed concluding that, *in vivo,* OCP seems more bioactive than other calcium phosphate phases. Using the present invention method, DCPD, OCP, HA and combination thereof can be deposited by controlled precipitation on metal implants.

Preferred embodiments of the present invention are alone or in combination mentioned in the following:
a) the Sub-process-2, 3 and 4 of Step-1 are performed, Sub-process-1 is dismissed;
b) the Sub-process-2 of Step-1 is performed under hydrothermal conditions;
c) the Sub-process-1, 2 and 3 of Step-1 are performed, Sub-process-4 is dismissed;
d) the Sub-process-2 of Step-1 is carried out using a solution comprising NaOH or KOH or H₂O₂ or combination thereof;
e) the Sub-process-3 of Step-1 is carried out under controlled atmosphere comprising inert gas or reducing gas;
f) the Ca²⁺ activity during Step-2 is actively controlled according to a defined profile; and
g) the implant is a non-Ti or non-Ti-alloy and the chemical Sub-process-2 and Sub-process-3 make use of acidic solutions.

Preferred embodiments of the present invention are hereinafter described in more detail with respect to the attached drawings which depict in:
Figure 1 - SEM micrographs: (A) grafting layer obtained on machined Ti surface and after Step-1. (B) The same sample after coating with OCP (Step-2) with its typical nano-lamellar morphology; and
Figure 2 - SEM micrographs after FIB milling: (A) Machined Ti surface after step 1&2; (B) Sample after HA deposition (Step-2) without grafting layer (Step-1); and
Figure 3 - SEM micrographs of blasted dental implants and after Step-1 and different conditions applied for Step-2.

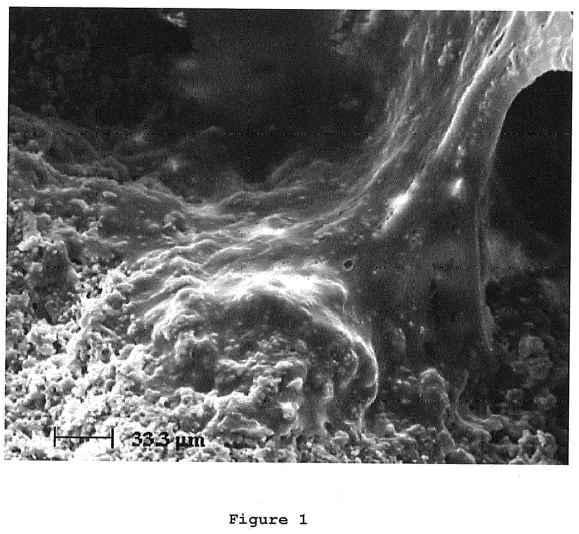

The method provides an efficient protocol for biomimetic coatings of metal (e.g., Ti and Ti-alloy) implants with CaPc. The obtained control over the precipitation process has been achieved because of several years of research focused on the theoretical study of solid phases' formation from solution with the aim to disclose the details of the precipitation mechanism. Such studies were developed on amorphous calcium carbonate (ACC), which is a model system, on calcium silicate hydrate (CSH), which is the main constituent of cements, and on CaPc phases. Thanks to these theoretical and experimental studies on the nucleation and growth mechanism of such materials and the development of the thermodynamic-kinetic models for their formation from solution, the method was fine-tuned to predict the exact experimental conditions at every time step of the CaPc formation (e.g., temperature, pH, ionic strength, concentration of all chemicals, and speed of adding such chemicals into a defined reactor) to promote heterogeneous nucleation and, then, growth of a layer of the target ceramic material on the metallic surface.
To evaluate the *in vitro* performance of the coated materials, the method can be applied to a polished Ti Grade 4 disks. The samples were treated according to the mentioned two-steps process. The first step (Step-1) comprises a sequence of sub-processes: the metal surface to be treated may undergo several mechanical, electrochemical, chemical, and thermal processes to obtain a rough and highly porous surface with appropriate grafting features (see Figure 1A, grafting layer). A preferential first sub-process comprises a mechanical (such as blasting) or electrochemical treatment on the machined surface to generate a primary roughness in the range of few micrometers. A preferential second sub-process comprise a chemical treatment with an alkaline solution (which include but it is not limited to NaOH, KOH, H₂O₂, and combination thereof) at temperature between 25 and 400 °C, pressure between 1 and 300 bar, and time between 10 minutes and 1 week, in order to generate an appropriate chemical phase on the surface of the metal implant. A preferential third sub-process comprises a thermal treatment in the oven under controlled atmosphere in the temperature range between 100 and 1000 °C and for the time between 0.1 to 48 h to promote the phase transformation of the chemical phase formed during the second sub-process and an appropriate metal-ceramic joining of the grafting layer. A preferential fourth treatment comprises a chemical treatment with an alkaline solution in order to improve the reactivity the thermally treated grafting layer. Thus, Step-1 includes fourth sub-processes, and it is substantially different with respect to the procedure described by Kokubo et al. (T. Kokubo, S. Yamaguchi, (2010), Bioactive Ti Metal and its Alloys Prepared by Chemical Treatments: State-of-the-Art and Future Trends, Advanced Biomaterials, 12, B579).

The grafting layer can be obtained according to the described procedure on Ti and Ti-alloy, but the same functional properties of such grafting layer can be achieved on other metals and metals alloy using a modified protocol which can comprise the use of acidic solution in the second and fourth Sub-processes.

In the second step (Step-2), the sample is immersed into a coating reactor for controlled CaPc precipitation. The coating is done under controlled conditions in order to obtain the desired calcium phosphate phase(s), comprising hydroxyapatite (HA), octacalcium phosphate (OCP), tricalcium phosphate (TCP), amorphous calcium phosphate (ACP), dicalcium phosphate dihydrate (DCPD), dicalcium phosphate anhydrous (DCPA), and combination thereof, with controlled porosity, morphology, thickness, and deposition rate. In the given example of Figure 1B, the experimental conditions were selected to promote the formation of OCP. The ceramic phase was confirmed by X-ray diffraction and Raman spectroscopy. In the applied experimental conditions, OCP has a delicate nanomorphology, which consists of lamellas with a thickness of few nm and an equivalent diameter of few microns. The coating method is able to provide a CaPc layer in aqueous media by controlling the nucleation and growth process of the targeted ceramic phase(s). The layer grows into and onto the grafting layer which is strongly joined the metallic implant.

The solid phase formation is promoted by controlling the saturation ratio of the system. The saturation ratio can be controlled in a different way comprising the addition of calcium ion solution into a phosphate ions solution where the parts to be coated are immersed. The saturation ratio can be kept either constant during the coating procedure or increased or decreased according to the desired coating protocol in order to obtain the appropriate gradient ceramic properties comprising morphology, phase, thickness, adhesion, and the optimal deposition rate.

The concentration of the calcium and phosphate ion solutions can be in the range 1-500 mM, more specifically in the range 5-50 mM, and the pH in the range 6-10 more specifically in the range 7-9, more specifically close to the physiological pH (7.4 ± 0.5). The Ca²⁺ solution may contain other cations comprising, for instance, Mg²⁺ or Sr²⁺.

The phosphate solution may contain other anions comprising for instance carbonate and/or fluorine ions and/or organic carboxylic acid.

The temperature is kept in the range 5-95 °C, more specifically in the range 25-80 °C, more specifically in the range close to the physiological temperature (37±5 °C).

The ionic strength can be in the range 0.002 - 0.5 M, more specifically in the range 0.01-0.2 M, more specifically close to the human isotonic condition (0.154 ± 0.05 M).

The calcium ion solution is added into the phosphate solution according to a defined flow rate in the range which depends on the reactor volume. The ratio between the flow rate (in L/h) and the reactor volume (in L) can be in the range 0.005 - 0.5 h⁻¹, more specifically in the range 0.05 ± 0.005 h⁻¹.

The gas atmosphere in the reactor can be controlled in term of temperature, humidity, and composition. Typically, air or N₂ stream, CO₂-free, water-saturated, and at the same temperature set of the Step-2, is used.

Temperature, pH, ionic strength, calcium ion solution flow rate, and calcium ion activity can be kept either constant during the entire coating process or varied according to a predefined protocol or actively controlled during the coating process.

The coating time, which depends on the specific experimental conditions, the calcium phosphate phase targeted, and the wanted thickness, can be in the range 0.1-24h, more specifically 1-5h, more specifically 1-2h.

The saturation level during the coating process with respect to the solubility of the formed ceramic phase is controlled by the deposition conditions and can be in the range 1-50, more specifically in the range 1-10. The saturation level is evaluated by a complex kinetic-thermodynamic model, which is calculating the activities of all known chemical species in solutions comprising:
OH⁻, Ca²⁺, PO₄³⁻, H₂PO⁴⁻, H₃PO₄, CaHPO₄, CaPO₄⁻, H⁺,
CaH₂PO₄⁺, P₂O₇⁴⁻, HP₂O₇³⁻, H₂P₂O₇²⁻, H₃P₂O₇⁻, H₄P₂O₇, HPO₄²⁻, M⁺, X⁻, clusters such as Caₓ(PO₄)_{y}(OH)_{z}(H)ₖ(M)ₘ(X)ₙ^{h+}, MHPO₄
where M is a cation including - but it is not limited to-Na⁺, K⁺, NH₄⁺ and X an anion including - but it is not limited to - Cl⁻, NO₃⁻, F⁻
and the solid phase(s) formed including HA, OCP, TCP, ACP, DCPD, and DCPA.

In a typical Step-1 procedure, a blasted metal implant is immersed in NaOH or NaOH/H₂O₂ solution and soaked for 12 to 24h at 40 to 90 °C, while the part is kept under constant rotation in the static or mixed alkaline solution. Alternatively, a blasted metal implant is treated with KOH or KOH/ H₂O₂ solution in an autoclave for 0.5 to 6 h at 100 to 250 °C. The chemically treated implant is washed and treated in a static oven in air for 0.5 to 6h at 400 to 900 °C. Alternatively, the chemically treated and washed implant is treated in an oven with controlled flowing atmosphere (inert or reducing atmosphere) for 0.5 to 6h at 200 to 700 °C. The thermally treated implant is soaked in a solution containing a phosphate buffer. Alternatively, the thermally treated implant is immersed in an alkaline solution for 0.5 to 6h at 25 to 90 °C.

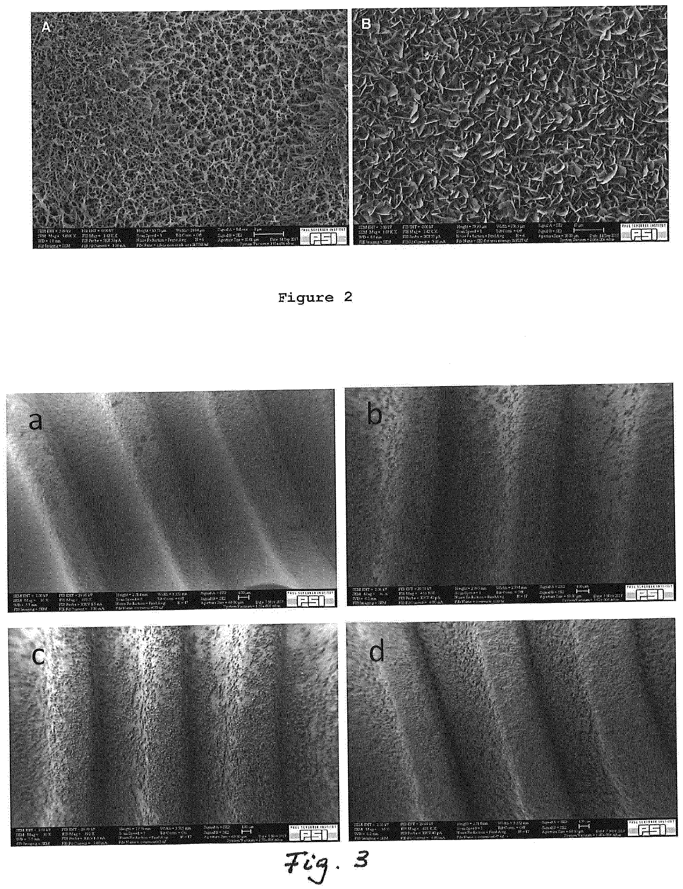

In a typical Step-2 procedure, the implant treated according to Step-1 is soaked in a reactor containing a phosphate solution, which is kept under stirring. Preferentially, the implant is kept under constant rotation during the entire Step-2 procedure. The position of the implant into the reactor can be optimized base on fluid dynamic modelling in order to achieve an optimal coating homogeneity. The coating may be triggered by addition of Ca²⁺ solution into the reactor. The coating process is monitored by online data collection, that is performed using a commercial analytical setup composed by titrator devices equipped with dosing burettes and anti-diffusion tips, pH electrodes equipped with Pt1000, ion-sensitive electrodes, and in-situ Raman spectroscopy. The ionic strength evolution is controlled online by a conductivity module. The saturation level is evaluated by means of the signals generated by the ion-sensitive electrodes and computed by means of the thermodynamic-kinetic model for the precipitation process. The saturation level is related to the driving force of the deposition and needs to be kept in a range where nucleation and growth occur only on the surface to be coated. Thus, during the entire Step-2 the activity of Ca²⁺ ion into the reactor is monitored and actively controlled. The activity of Ca²⁺ can be kept constant, increased or decreased during the entire Step-2 in order to optimize the coating in term of CaPs phase, thickness, porosity, morphology, and deposition rate. An optimal coating protocol is defined by a specific Ca²⁺ activity profile during the entire Step-2 procedure. The coating method can be easily scaled up to coat simultaneously several Ti implants immersed in a large reactor.
In a simplified lab-scale procedure, a 10 mM CaCl₂ solution is added at a constant rate (30 µL/min) in a 0.035 L of a 10 mM NaH₂PO₄/Na₂HPO₄ buffer, kept under constant stirring and temperature (37 °C) in a double-jacket pyrex-glass vessel connected with an external thermostatic bath. The pH is kept constant at 7.4 via counter titration of alkaline (NaOH, 10 mM) and acidic (HCl, 10 mM) solutions. The ionic strength is kept constant at 0.154 M by NaCl addition. The solid calcium phase formation is followed by the ion-sensitive electrodes and the coating time is of 2h. The coated parts are removed from the reactor and rinsed with milliQ water. In such experimental conditions, a layer of 3-5 µm of OCP phase is deposited on the metallic support. The layer is composed by lamellar calcium phosphate. Each lamella has a thickness of about 2-5 nm and an average size of 2-5 µm. The ceramic phase grew in the porosity of the grafting layer and on top of it. The ceramic layer is highly porous and strongly joined with the metallic support. Figure 2A shows a scanning electron microscopy (SEM) cross section of the sample, obtained by focused ion beam (FIB) milling. The coating time of such sample was about 12h; as a result, it was overcoated with OCP. In those specific experimental conditions, 1-2h treatment is sufficient to reach an appropriate coating thickness. As a comparison, Figure 2B shows the result of OCP deposition on a polished Ti metal surface (Step-2 without Step-1): OCP is anyway deposited, but the ceramic layer is clearly delaminated at the metal-ceramic interface because an appropriate grafting layer is missing. Thus, the not-properly deposited CaPc layer can easily delaminate once implanted.

Figure 1 shows SEM micrographs: (A) grafting layer obtained on machined Ti surface and after Step-1; (B) reports on the same sample after OCP coating (Step-2) with its typical nano-lamellar morphology.

Figure 2 shows SEM micrographs after FIB milling. Figure 2(A) refers to a machined Ti surface after step 1&2. On the Ti substrate, first a dense layer (about 120 nm) of Ti-based material and then a porous layer (about 1 µm) of Ti-based material are formed by chemical and thermal treatments. After Step-2, most of the porosity of the grafting layer was filled with OCP. On top, a thick but open-porous layer of several microns of OCP was grown. Figure 2(B) shows the same sample after OCP deposition (Step-2) without grafting layer (Step-1). Thus, OCP was grown directly on a machined Ti surface. The absence of a grafting layer induces delamination of the CaPc layer from metal. In the inset, the consequent extended fracture at the metal-ceramic interface is shown. The chemical composition of each layer was identified by EDX analysis.

Figure 3 shows SEM micrographs of dental implants treated according Step-1 and different Step-2 treatment in order to achieve different coatings. In Figure 3a, only a very small amount of CaPc is deposited inside the porous structure of the grafting layer. In Figure 3a-d, a progressively higher amount of OCP is deposited (0.5, 2, 4, 8h, respectively).

## Claims

1. A method for biomimetic growth of CaPc on a metal implant, particularly on a Ti and Ti-alloy implant, comprising the following steps:
Step-1: a combination of treatments in order to generate an appropriate grafting layer on the metal implant; such Step-1 is preferably composed of one or more of the following Sub-process:
i) Sub-process-1: a mechanical or electrochemical treatment on the machined surface in order to generate a primary roughness in the range of few micrometers; and/or
ii) Sub-process-2: a chemical treatment with an alkaline solution, in order to generate an appropriate chemical phase on the surface of the metal implant; and/or
iii) Sub-process-3: a thermal treatment in order to promote an appropriate metal-ceramic joining of the grafting layer; and/or
iv) Sub-process-4: a final chemical treatment with an alkaline solution in order to improve the reactivity grafting layer; and
Step-2: coating of the implant treated according to Step-1 by controlled CaPc precipitation.

2. The method according to claim 1 wherein the Sub-process-2, 3 and 4 of Step-1 are performed.

3. The method according to claim 1 or 2 wherein the Sub-process-2 of Step-1 is performed under hydrothermal conditions.

4. The method according to claim 1 wherein the Sub-process-1, 2, and 3 of Step-1 are performed.

5. The method according to any of the preceding claims, where the Sub-process-2 of Step-1 is carried out using a solution comprising NaOH or KOH or H₂O₂ or combination thereof;

6. The method according to any of the preceding claims, where the Sub-process-3 of Step-1 is carried out under controlled atmosphere comprising inert gas or reducing gas.

7. The method according to any of the preceding claims, where the Ca²⁺ activity during Step-2 is actively controlled according to a defined profile.

8. The method according to any of the preceding claims where the implant is not Ti or Ti-alloy and the chemical Sub-process-2 and Sub-process-3 an acidic solutions might be used.
